(19) Europäisches Patentamt

European Patent Office

Office européen des brevets

(11) **EP 1 532 926 A1**

(12) **EUROPEAN PATENT APPLICATION**

(43) Date of publication:
**25.05.2005 Bulletin 2005/21**

(51) Int Cl.⁷: **A61B 5/16**

(21) Application number: **04257178.6**

(22) Date of filing: **19.11.2004**

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: **20.11.2003 JP 2003391360**

(71) Applicant: **SONY CORPORATION**
**Tokyo (JP)**

(72) Inventors:
  • **Asukai, Masamichi, c/o Sony Corporation**
    **Tokyo (JP)**
  • **Sako, Yoichiro, c/o Sony Corporation**
    **Tokyo (JP)**
  • **Terauchi, Toshiro, c/o Sony Corporation**
    **Tokyo (JP)**
  • **Inoue, Makoto, c/o Sony Corporation**
    **Tokyo (JP)**
  • **Shirai, Katsuya, c/o Sony Corporation**
    **Tokyo (JP)**
  • **Miyajima, Yasushi, c/o Sony Corporation**
    **Tokyo (JP)**
  • **Makino, Kenichi, c/o Sony Corporation**
    **Tokyo (JP)**
  • **Takai, Motoyuki, c/o Sony Corporation**
    **Tokyo (JP)**

(74) Representative: **Nicholls, Michael John**
    **J.A. KEMP & CO.**
    **14, South Square**
    **Gray's Inn**
    **London WC1R 5JJ (GB)**

(54) **Emotion calculation apparatus and method**

(57) In a method and an apparatus for calculating the emotion of the human being, the emotion of a user of the apparatus is calculated from the pressure acting on an object. A pressure sensor 22 is provided in a mobile phone 20. The pressure sensor 22 detects the pressure with which the user grips the mobile phone and the pressure exerted by the user in key inputting. An emotion calculating unit 23 calculates emotional data of the user based on the pressure detected by the pressure sensor 22. The emotion data is a value pertinent to affect-induction as one dimension of an emotional model. The mobile phone feeds the emotion calculated to a user or to a counterpart party to the user or to a counterpart party of communication.

FIG.1

EP 1 532 926 A1

**Description**

This invention relates to a method and an apparatus for calculating the emotion of the human being, and to a mobile communication apparatus for calculating the emotion for a counterpart party of communication or for the contents of communication.

This application claims priority of Japanese Patent Application No. 2003-391360, filed on November 20, 2003, the entirety of which is incorporated by reference herein.

In psychology, there are two theories for defining the emotion, that is, the fundamental emotion theory and the dimensional theory. The fundamental emotion theory is presupposed on the notion that the emotion has been evolved and hereditarily incorporated to meet the needs such as those met for the existence of the living body, and that the living body has, by nature, the fundamental emotion of 'surprise', 'anger', 'disgust', 'sadness' and 'happiness'.

The dimensional theory, on the other hand, does not handle the emotion discretely, as does the fundamental emotion theory, but expresses the emotion as a vector on continuous dimensions, having the emotion values on plural axial directions. Suppose that a vial is placed here, and that the vial has variegated patterns. If this vial is seen from different directions, it appears in variable fashions. However, the vial is a sole entity and is, after all, the same vial, even though it is viewed from different directions. As may be inferred from the instance of this vial, the emotion may be grasped differentially depending on the viewing angle, that is, depending on the different context or situation, such that the emotion may appear as totally different emotion. That is, the emotion 'anger' does not have a specified constant pattern and simply a certain state having a certain direction and magnitude as a vector is labeled 'anger', and may be recognized to be a different feeling, such as 'fear', depending on the particular context or situation. This is the basic concept of the dimensional theory.

With the dimensional theory, a variety of coordinate spaces, termed emotional models, has been proposed for expressing the emotion. As one of the emotional models, the Levin's emotional model has the arousal and valence as two emotional directions. The valence is a concept of the dynamic psychology, and is relative to positive and negative properties the human being has for a subject. The human being is attracted towards an object having positive valence and evades a subject having negative valence. In general, if the subject has high valence, the user's emotion may be labeled 'happiness', 'relaxation' and 'amenity', If conversely a subject has low valence, the user's emotion may be labeled 'sadness', 'boredom', 'fear' or 'stress'. The valence may be measured in accordance with the relationship between the valence 'Sentics' shown by Clynes and the pressure (see Non-Patent Publication 1).

[Non-Patent Publication 1] Clynes, M "Sentics: Biocybernetics of Emotion Communication", Annals of the New York Academy of Science, 220, 3, 55-131, 1973.

It is an object of the present invention to provide a method and an apparatus for calculating the emotion of a user from the pressure the user applies to an object, and to a mobile communication apparatus.

In one aspect, the present invention provides an emotion calculating apparatus comprising pressure detection means for detecting the pressure exerted on an object from a user, and emotional data calculating means for calculating emotional data, indicating the level of the affect-induction, based on the pressure detected by the pressure detection means. The emotional data is a value specifying the affect-induction which is one direction of the emotion.

A mobile communication terminal according to the present invention outputs the user's emotion, calculated by the emotional data calculating unit, to an external communication apparatus. The mobile communication terminal memorizes emotional data and a communication apparatus, with which the mobile communication terminal was communicating when the emotional data was calculated. The communication counterpart notifying means outputs a ring tone or an incoming display image surface, indicating who is the counterpart party of communication, based on past emotional data stored in the emotional data storage means.

An emotion calculating method according to the present invention comprises a pressure detection step for detecting a pressure exerted by a user on an object, and an emotional data calculating step of calculating emotional data, based on a pressure exerted by a user on the object.

According to the present invention, the user's emotional data may be calculated based on the pressure exerted by a user on an object. By combining the emotional data with the counterpart party with whom the user was communicating when the emotional data was calculated, it is possible to learn the unconscious emotion the user entertains for the counterpart party of communication and for the contents of communication. Moreover, the expression of the information may be made richer by putting emotional data into the contents of the communication.

Embodiments of the invention will now be described by way of non-limitative example with reference to the accompanying drawings, in which:

Fig. 1 is a block diagram showing a basic configuration of the present invention.

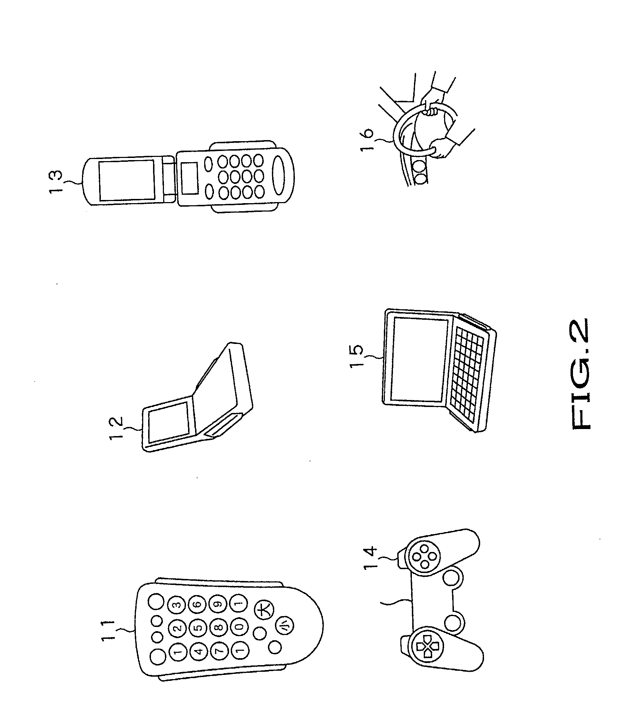

Fig.2 shows an illustrative mounting position for a pressure sensor.

Fig.3 is a block diagram showing an inner structure of a mobile communication apparatus embodying the present invention.

Fig.4 shows an illustrative mail sentence.

Fig.5 shows the structure of a database.

Fig.6 is a flowchart showing the operation of a mobile phone during call with speech.

Fig.7 is a flowchart showing the operation of a mobile phone during mail transmission/ reception.

Fig. 8 is a block diagram showing an inner structure of a game machine embodying the present invention.

According to the present invention, the relationship between the pressure exerted from the user to an object and affect-induction is used in calculating the user's emotion. This affect-induction, which is a concept of dynamic psychology, is a property of attraction of an object exerted on a person or a property of causing a person to evade an object. If an object attracts a man, such object is termed an object having positive affect-induction and, if an object entices a man to avoid it, such object is termed an object having negative affect-induction.

It is generally accepted that the higher the affect-induction of an object, the higher is the goodwill or. interest a man has in the object, and that, conversely, the lower the affect-induction of an object, the lower is the goodwill a man has in the object and the lesser is the interest a man has in the object. In the present invention, data indicating the level of the affect-induction is termed emotional data.

In the present invention, an object acted on by a user is termed a subject of operation 1. An object which attracts a user when the user acts on the subject of operation 1 is e.g. the subject of operation 1 itself or the environment surrounding the user acting on the subject of operation 1. For example, when the user is acting on a mobile phone, the contents of talk of the mobile phone or the contents of the mail being prepared is an object which attracts the user. When the user is driving a car, the music furnished in the car or the scene viewed from inside the car is an object which attracts the user.

According to the present invention, a pressure sensor 2 is provided to the subject of operation 1, and emotional data of a user for the subject of operation is calculated based on an output of the pressure sensor 2. The affect-induction is utilized for operational control of the electronic equipment.

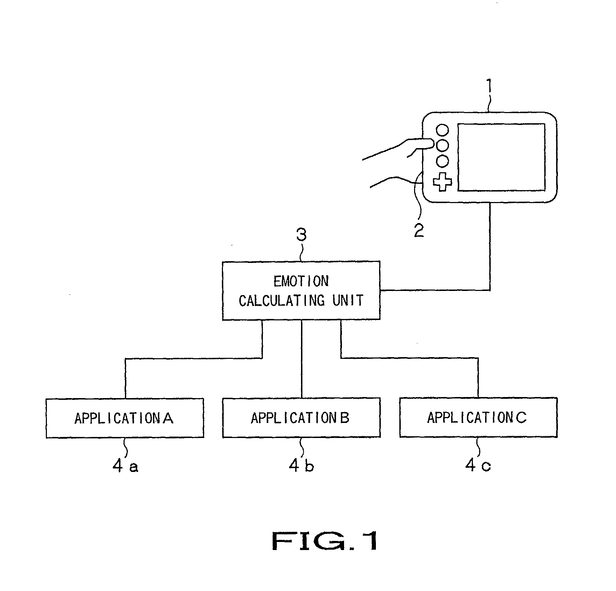

Fig.1 shows basic constituent elements of the present invention. The present invention comprises the pressure sensor 2 for detecting the pressure acting on the subject of operation 1, an emotion calculating unit 3 for calculating the level of affect-induction, based on the user's pressure, and application units 4a to 4c for executing the processing in keeping up with the user's emotion.

The pressure sensor 2 is provided to the subject of operation 1. The subject of operation 1 may be enumerated by a mobile phone, a PDA (Personal Digital Assistant), a remote controller, a game controller, a hand-held computer, and a handle of a vehicle.

The subject of operation 1 is shaped to permit the user to hold it with one or both hands. The user operates the subject of operation 1 as he/she holds it with a hand. The pressure acting on the subject of operation 1 from the user's hand is detected by the pressure sensor 2. The pressure sensor 2 may be enumerated by a high molecular piezoelectric film, the capacitance of which is changed e.g. by bending a film, piezoelectric rubber, the resistance of which is changed by pressure, and a strain gauge, the resistance of which is varied minutely on strain generation. The pressure sensor 2 may be in the form of a surface conforming to the surface of the subject of operation 1 or in the form of a dot at the center of a virtual grating provided on the surface of the subject of operation 1.

The pressure sensor 2 may be provided only on a portion of the subject of operation 1 contacted with the user's hand. For example, in the case of a mobile phone 13, the root of a user's thumb finger and the inner sides of the user's fingers except the thumb finger are contacted with both sides of the mobile phone 13. Hence, the pressure sensor is provided in each of these positions. The subject of operation 1, in which plural pressure sensors are provided on sites similar to those of the mobile phone 13, may be enumerated by a remote controller 11 and a PDA 12. These subjects of operation 1 are shaped so as to be held with the user's one hand.

With the subject of operation 1 of the type held with both hands, such as a game controller 14 or a hand-held computer 15, the user's hand contacts with both sides of the subject of operation 1. Hence, the pressure sensors 2 are provided on similar locations of the subject of operation 1 of the type held with both hands. The number of the pressure sensors 2 may be decreased by providing the pressure sensors only on the portions of the subject of operation contacted by the user's hand.

The pressure sensors 2 are also provided on an input section 5 of the subject of operation 1. For example, the input button may be physical button or a virtual button demonstrated on an image display surface. The physical input button may be enumerated by a slide key or a cross-shaped key indicating the direction, in addition to a toggle key for inputting the binary information. These buttons are provided to the PDA 12, remote controller 11 or to the game controller 14 as well.

The application units 4a to 4c process the electronic equipment, responsive to emotional data calculated by an emotion calculating unit. The application units 4a to 4c identify a subject in which a user feels affect-induction. The subject in which a user feels affect-induction is e.g. the game contents in a game controller 14, contents of a television or video in a remote controller 11, the music provided during driving, or states of a road in a handle 16 of a vehicle. The application units 4a to 4c exploit the affect-induction for the subject of operation 1 for operational control of the electronic equipment. An instance of such exploitation is hereinafter explained.

[Example 1]

In this Example 1, the present invention is applied to a mobile phone 20. This mobile phone 20 calculates the affect-induction for a counterpart party of communication, and feeds the user's unconscious feeling back to the user or to the counterpart party of communication.

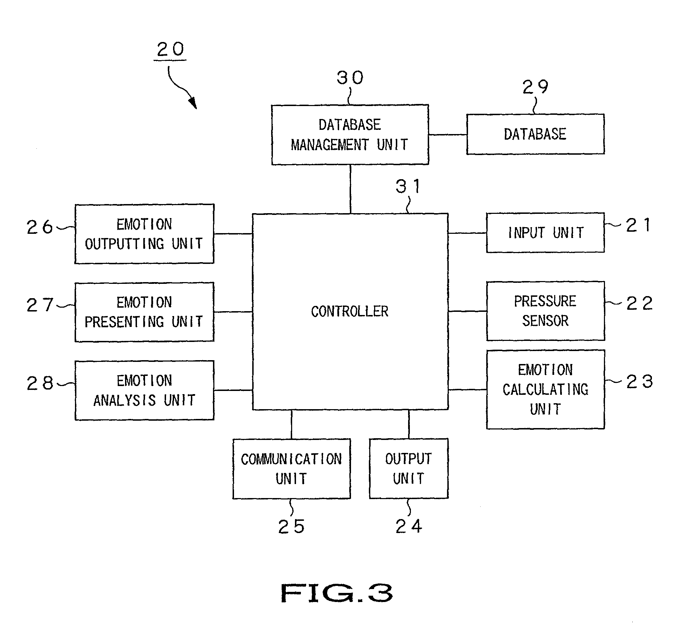

Fig.3 is a block diagram showing an inner structure of the mobile phone 20. The mobile phone 20 includes an input unit 21, a pressure sensor 22 for detecting the gripping pressure applied to a main body unit or to the input unit 21, an emotion calculating unit 23 for verifying likes and dislikes or degree of depth of interest for the counterpart party or the contents of communication, an output unit 24 for outputting the speech or the image, a communication unit 25 for information exchange or modulation/ demodulation of the electrical waves, an emotion outputting unit 26 for advising the user of the emotion the user entertains for the counterpart party of communication, an emotion presenting unit 27 for advising the counterpart party of communication of the emotion the user entertains for the counterpart party or the contents of communication, a database 29 for storing the emotion, and a database management unit 30 for supervising the storage contents of the database. The mobile phone 20 also includes an emotion analysis unit 28 for performing statistic processing on the results stored in the database 29 to advise the user of the results of the statistic processing, and a controller 31 for controlling the mobile phone 20 in its entirety.

The emotion calculating unit 23 calculates the contents of communication of the mobile phone 20 and the affect-induction for the counterpart party of communication. The affect-induction and the pressure applied by the user are correlated, such that, the higher the affect-induction, the higher becomes the pressure. The relationship between the affect-induction and the pressure is not a monotonous increase, but there exist several exceptions. For example, even if the pressure exerted by the user is high, but the pressure is applied only instantaneously, this pressure represents not the goodwill but 'anger'. The emotion calculating unit 23 processes such exception in a particular fashion.

The emotion outputting unit 26 identifies the counterpart party of communication and advises the user of the feeling for the counterpart party of communication. The emotion outputting unit 26 outputs the sound, light, image or the vibrations to transmit the emotion. The emotion transmitted to the user may be the user's current emotion or the user's past emotion stored in a database.

The emotion presenting unit 27 advises the counterpart party of communication of the user's emotion. The emotion presenting unit 27 converts the user's emotion into e.g. the sound, light, letters or characters, images, pictures or vibrations to output these to the counterpart party of communication. Specifically, the emotion presenting unit 27 varies the ring sound, incoming image display surface, light emitting patterns of the light emitting device, or the vibration pattern of the vibrator, on the part of the counterpart party of communication.

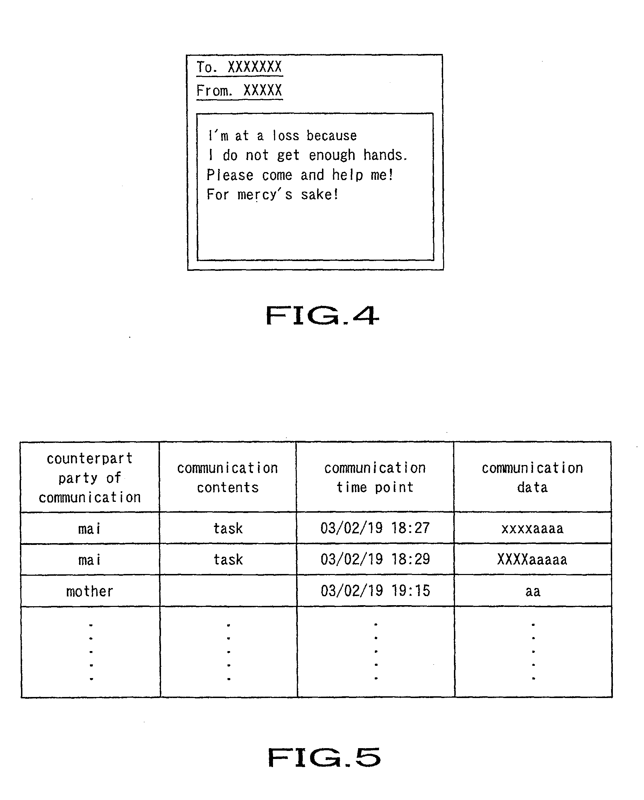

The emotion presenting unit 27 puts the user's feeling in the contents of a mail. A mail shown in Fig.4 states a sentence: "I'm at a loss because I do not get enough hands. Please come and help me! For mercy's sake!". In the mail prepared by the emotion presenting unit 27, the letters for "Please come and help me!" and "For mercy's sake!" are larger in size. This is because the user's affect-induction is high when the user stated "Please come and help me!" and "For mercy's sake!" and hence the letter size is made larger in order to emphasize the letters. The emotion presenting unit 27 varies the color or thickness of the letter, color or the pattern of the background. The emotion presenting unit 27 also inserts pictograms or facial letters conforming to the user's emotion.

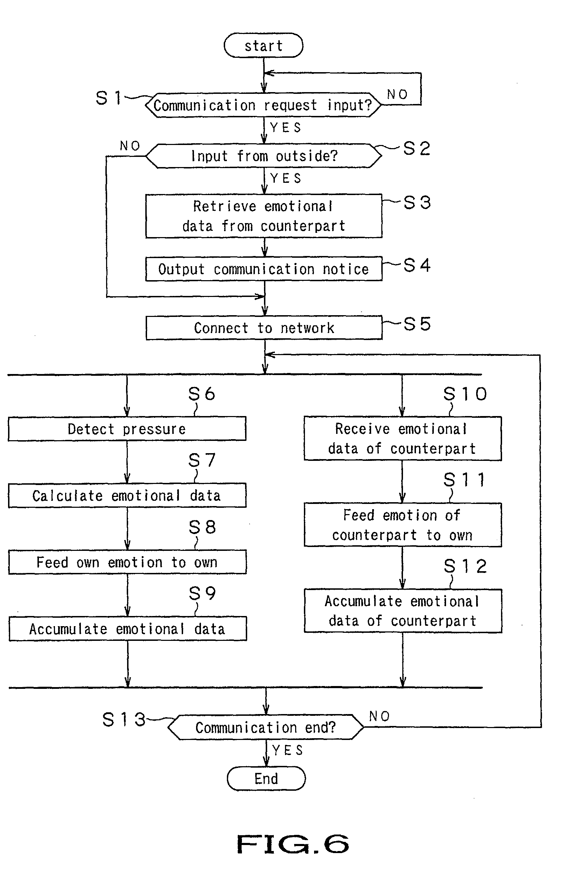

The database 29 classes the calculated results of the emotion calculating unit 23 from one counterpart party of communication to another. The database 29 memorizes the counterpart party of communication, contents of communication, emotional data or the date/ time of communication, as shown in Fig.5. The database management unit 30 receives the information pertinent to the counterpart party of communication from the communication unit 25, while being supplied with the emotional data from the emotion calculating unit 23. The database management unit 30 also correlates input emotional data with the counterpart party of communication for storage of the so correlated data in the database 29.

The emotion analysis unit 28 analyzes the emotion data recorded on the database 29. The emotion analysis unit 28 performs statistic processing on emotional data to verify the tendency of change in the user's emotion, or outputs the change in the emotion as a diagram in the database 29.

Referring to Fig.6, the processing sequence in outputting the emotion a user entertains for a counterpart party of communication or the emotion the counterpart party of communication entertains for the user, in the course of call over the mobile phone 20, to the user, is now explained.

The mobile phone 20 awaits a communication request from an external base station or from a user. On receipt of the communication request from the mobile phone 20 (step S1, YES), the mobile phone 20 verifies whether or not the communication request is from a user or from an external counterpart party of communication (step S2). In case the mobile phone has received a communication request from an external (step S1, YES). On receipt of a communication request from the external counterpart party of communication (step S2, YES), the mobile phone 20 identifies the counterpart party of communication, and retrieves emotion data concerning the counterpart party of communication from the database 29 (step S3). The emotion outputting unit 26 is responsive to the emotional data retrieved to output the ring tone, incoming image surface, light emitting patterns of the light emitting device or the vibrating pattern of the vibrator (step S4). When the user's mobile phone 20 responds to the ring tone, the mobile phone 20 proceeds to make network connection with the counterpart party of communication (step S5).

When the user has requested communication (step S2; NO), the mobile phone 20 proceeds to make network connection with the counterpart party of communication specified by the user (step S5). Lacking the communication request in the step S1 (step S1; NO), the mobile phone 20 awaits the generation of the communication request.

When the connection with the external counterpart party of communication is established, the mobile phone 20 proceeds to perform the processing of feeding back the emotion for the counterpart party of communication to itself (steps S6 to S9) and the processing of feeding back the emotion the counterpart party of communication entertains for the user to the user's self (steps S 10 to S12).

First, the processing of feeding the emotion for the counterpart party of communication to the user' self is explained. The pressure sensor 22 detects the pressure with which the user grips the mobile phone 20 or the pressure applied by the user on the input button is detected (step S6). The emotion calculating unit 23 calculates emotional data e from the pressure P exerted by the user on the mobile phone 20 or the user's force of pressure on the mobile phone 20. The emotional data e may be calculated from the following equation (1):

$$e = P/Pmax$$

where Pmax is the maximum value of the pressure. The equation (1) normalizes the pressure P. Meanwhile, the denominator on the left side of the equation (1) may be an average value or the standard deviation of the pressure (step S7).

The emotion outputting unit 26 advises the user of the emotional data e, calculated by the emotion calculating unit 23. The medium used in notifying the emotional data e is e.g. the sound, light, image, picture or vibrations. In notifying the emotional data e, the emotional data e may be output as numerical values, or in different forms of expression for the emotion. Or, the value of the emotional data may be expressed by outputs of the light emitting device (step S8).

The database management unit 30 then identifies the user's counterpart party of communication and proceeds to store the emotional data e in the database 29 in association with the counterpart party of communication (step S9).

The processing of feeding the emotion entertained by the counterpart party of communication for the user to the user's self is explained. The mobile phone 20 receives emotional data of the counterpart party of communication (step S10). The mobile phone 20 outputs the received emotional data in the form of sound, light, image, picture, vibrations or letters/ characters.

By simultaneously outputting the emotion entertained by the counterpart party of communication for the user and the emotion entertained by the user for the counterpart party of communication, the mobile phone 20 is able to notify the compatibility of temperament between the user and the counterpart party of communication and the degree of matching of the opinion between the user and the counterpart party of communication (step S11).

The database management unit 30 stores the emotional data of the counterpart party of communication for the user, as received in the step S 10, in the database 29 (step S12). The emotion analysis unit 28 maps the emotional data, stored in the database 29, in a graphical form, or analyzes the emotional data.

When the emotional data has been stored, the mobile phone 20 verifies whether or not the speech has come to an end (step S 13). When the call is going on (step S 13; NO), the mobile phone 20 proceeds to the processing in a step S6. If the call has come to a close (step S 13; YES), the mobile phone 20 finishes the processing.

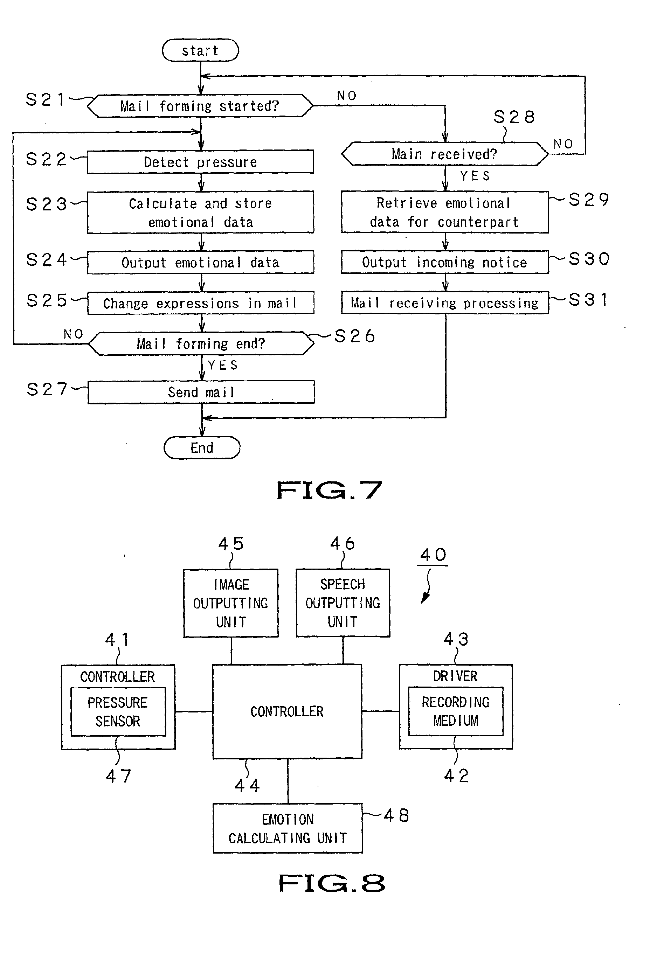

The processing in outputting the user's feeling to the counterpart party of communication, when transmitting/ receiving a mail, is now explained in accordance with the flowchart of Fig.7.

The mobile phone 20 awaits receipt of a mail from outside or start of preparation of a mail by the user. When the user commences to prepare a mail (step S21; YES), the pressure exerted by the user's hand on the mobile phone 20 is detected (step S22). The emotion calculating unit 23 calculates emotional data from the pressure to store the so calculated emotional data in the database 29 (step S23). The emotion outputting unit 26 demonstrates emotional data on an image display surface or emits light expressing the emotion. From these outputs, the user is able to know the own unconscious emotion for the counterpart party of communication or the contents of the mail (step S24).

When the preparation of a mail has come to a close, the emotion presenting unit 27 varies the size or color of the letters/ characters of the sentences or the color or the pattern of the background to put the user's emotion at the time of formulating the sentence in the mail. The emotion presenting unit 27 also varies the ring tone or the incoming image surface of the mail in order to advise the counterpart party of communication of the user's emotion for the counterpart party of communication (step S25). When the mail formulation has come to a close (step S26; YES), the communication unit 25 sends the mal formulated to the counterpart party of communication (step S27). When the mail formulation has not come to a close (step S26; NO), processing transfers to a step S22.

If the user is not formulating a mail (step S21; NO), and has received a mail (step S28; YES), the emotion outputting unit 26 retrieves past emotional data for the counterpart party of communication from the database 29 (step S29). The emotion outputting unit 26 then varies the ring tone or the incoming image surface based on the emotional data (step S30). The mobile phone 20 outputs the ring tone and subsequently receives the contents of a mail (step S31).

As described above, the mobile phone 20 according to the present invention detects the pressure with which the user grips the mobile phone 20 and that with which the user acts on the input button to calculate the emotion entertained by the user for the counterpart party of communication and for contents of communication, based on the so detected pressure.

By feeding the emotion the user entertains for the counterpart party of communication to the user's self, the user is able to become aware of his/her unconscious feeling for the counterpart party of communication. Moreover, by feeding the emotion the user entertains for the counterpart party of communication to the counterpart party of communication, the user's emotion can be transmitted emotion more profusely to the counterpart party of communication.

Additionally, since the mobile phone 20 is gripped for prolonged time, the pressure exerted by the user may be measured accurately.

In a second embodiment, the present invention is applied to a game machine 40. Referring to Fig.8, the game machine 40 includes a controller 41, as an inputting device, a storage medium 42 for storage of a game story, a driver 43 for reading out a game program, stored in a recording medium 42, a controller 44 for changing the process of the game story responsive to the user's input and the game program, an image outputting unit 45 outputting an image and a speech outputting unit 46 outputting the speech.

The controller 41 includes a pressure sensor 47. An emotion calculating unit 48 calculates the user's emotion based on the pressure applied from the user to the controller 41. The game story is changed based not only on the user's conscious input by the operation of the controller 41 but on the pressure generated unconsciously by the user at the time of the operation of the controller 41.

For example, if the user is deeply concentrated in the game, the story process of the game may be quickened. If the user's consciousness has deviated from the game, an event which attracts the user may be generated.

On the other hand, if the user's consciousness has suddenly deviated from the game, the game operation may be interrupted. Thus, even if an unforeseen event happened during the game operation, such as telephone call over or being called by a person, the game can be continued from a partway position.

The contents in which a user is interested may also be emphasized. For example, if the affect-induction of a user is raised when a certain character has appeared, the story process may be switched to a story development centered about the character.

**Claims**

1. An emotion calculation apparatus comprising:

   pressure detection means for detecting the pressure exerted from a user; and
   emotional data calculating means for calculating emotional data, indicating the level of the affect-induction, based on the pressure detected by said pressure detection means.

2. An emotion calculation apparatus according to claim 1 wherein said pressure detection means detects a pressure with which a user grips the emotion calculating apparatus.

3. An emotion calculation apparatus according to claim 1 or 2 further comprising:

   inputting means for a user to input an operating signal;
   said pressure detection means detects a pressure with which a user acts on said inputting means.

4. An emotion calculation apparatus according to any one of the preceding claims further comprising emotional data outputting means for converting the emotional data calculated by said emotional data calculating means into at least one of the sound, light, image, picture or vibrations, and for outputting resultant data

5. An emotion calculation apparatus according to any one of the preceding claims further comprising emotional data storage means for storing the emotional data calculated by said emotional data calculating means.

6. A mobile communication apparatus comprising:

   inputting means for a user to input an operating signal;
   an emotion calculation apparatus according to any one of claims 1, 4 or 5; and
   outputting means for outputting said emotional data.

7. A mobile communication apparatus according to claim 6 wherein said pressure detection means detects the pressure with which the user grips the mobile communication apparatus.

8. A mobile communication apparatus according to claim 6 wherein said pressure detection means detects the pressure with which the user acts on said inputting means.

9. A mobile communication apparatus according to any one of claims 6 to 8 further comprising:

electronic mail transmitting means for transmitting a sentence entered from said inputting means as electronic mail to external communication means; and
document changing means for changing the fonts of letters/ characters and/or the layout of the sentence stated in said electronic mail, based on the emotional data of the user.

10. A mobile communication apparatus according to any one of claims 6 to 9 further comprising emotional data storage means for storing the emotional data during communication and the identification information of a communication apparatus of the destination of communication.

11. A mobile communication apparatus according to any one of claims 6 to 10 further comprising:

emotional data retrieving means for retrieving emotional data on receipt of an electrical wave from another communication apparatus and emotional data at the time of past communication with said other communication apparatus, from said emotional data storage means; and
communication counterpart notifying means for notifying the emotion at the time of past communication by a ring tone and/or an incoming display image surface.

12. An emotion calculation method comprising:

a pressure detection step for detecting a pressure exerted by a user on an object; and
an emotional data calculating step of calculating emotional data, indicating the level of the affect-induction, based on a pressure exerted by a user on said object.

13. An emotion calculation method according to claim 12 further comprising an emotional data outputting step of converting the emotional data, calculated in said emotional data calculating step, into at least one of sound, light, image, picture and vibrations, and outputting resultant data.

14. An motion calculation method according to claim 12 or 13 further comprising an emotional data storage step of storing the emotional data, calculated in said emotional data calculating step, in emotional data storage means.

FIG. 1

FIG.2

EP 1 532 926 A1

FIG.3

```
To. XXXXXXX
From. XXXXX

┌─────────────────────────────┐
│ I'm at a loss because        │
│ I do not get enough hands.   │
│ Please come and help me!     │
│ For mercy's sake!            │
│                              │
└─────────────────────────────┘
```

# FIG.4

| counterpart party of communication | communication contents | communication time point | communication data |
|---|---|---|---|
| mai | task | 03/02/19 18:27 | xxxxaaaa |
| mai | task | 03/02/19 18:29 | XXXXaaaaa |
| mother | | 03/02/19 19:15 | aa |
| ⋮ | ⋮ | ⋮ | ⋮ |

# FIG.5

11

start

S1 — Communication request input? — NO

YES

NO — Input from outside? — S2

YES

Retrieve emotional data from counterpart — S3

Output communication notice — S4

Connect to network — S5

S6
Detect pressure

S7
Calculate emotional data

S8
Feed own emotion to own

S9
Accumulate emotional data

S10
Receive emotional data of counterpart

S11
Feed emotion of counterpart to own

S12
Accumulate emotional data of counterpart

S13 — Communication end? — NO

YES

End

FIG.6

start

S21 — Mail forming started? — NO

S22 — Detect pressure

S23 — Calculate and store emotional data

S24 — Output emotional data

S25 — Change expressions in mail

NO — Mail forming end? — S26

YES

S27 — Send mail

S28

Main received? — NO

YES

Retrieve emotional data for counterpart — S29

Output incoming notice — S30

Mail receiving processing — S31

End

## FIG.7

45 — IMAGE OUTPUTTING UNIT

46 — SPEECH OUTPUTTING UNIT

40

41 — CONTROLLER
PRESSURE SENSOR

47

CONTROLLER

44

EMOTION CALCULATING UNIT — 48

43 — DRIVER
RECORDING MEDIUM

42

## FIG.8

**European Patent Office**

# EUROPEAN SEARCH REPORT

Application Number

EP 04 25 7178

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>Y | WO 03/065893 A (TRAMITZ, CHRISTIANE, DR; BAYER, OTMAR) 14 August 2003 (2003-08-14)<br>* the whole document * | 1,2,4,5,<br>12-14<br>3,6-11 | A61B5/16 |
| Y | WO 02/17602 A (SYMBIAN LIMITED; RANDALL, STEPHEN; JENSON, SCOTT)<br>28 February 2002 (2002-02-28)<br>* the whole document * | 6-11 | |
| X | CLYNES M: "Sentography: dynamic forms of communication of emotion and qualities"<br>COMPUTERS IN BIOLOGY AND MEDICINE,<br>vol. 3, no. 2, September 1973 (1973-09),<br>pages 119-130, XP002317297<br>US<br>* the whole document * | 1-5,<br>12-14 | |
| E,X | EP 1 494 190 A (SONY CORPORATION)<br>5 January 2005 (2005-01-05)<br>* the whole document * | 1,12 | |
| Y | WO 03/036247 A (MICROJENICS, INC; NATIONAL INSTITUTE OF ADVANCED INDUSTRIAL SCIENCE; H) 1 May 2003 (2003-05-01)<br>* claim 9 *<br>& EP 1 447 653 A (MICROJENICS, INC; NATIONAL INSTITUTE OF ADVANCED INDUSTRIAL SCIENCE AN) 18 August 2004 (2004-08-18) | 3 | TECHNICAL FIELDS SEARCHED (Int.Cl.7)<br>A61B |
| A | US 5 507 291 A (STIRBL ET AL)<br>16 April 1996 (1996-04-16)<br>* the whole document * | 1-14 | |

The present search report has been drawn up for all claims

| Place of search | Date of completion of the search | Examiner |
|---|---|---|
| Munich | 11 February 2005 | Birkenmaier, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 532 926 A1

**ANNEX TO THE EUROPEAN SEARCH REPORT
ON EUROPEAN PATENT APPLICATION NO.**

EP 04 25 7178

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2005

| Patent document cited in search report | | Publication date | Patent family member(s) | | Publication date |
|---|---|---|---|---|---|
| WO 03065893 | A | 14-08-2003 | WO | 03065893 A1 | 14-08-2003 |
| | | | AU | 2002247698 A1 | 02-09-2003 |
| WO 0217602 | A | 28-02-2002 | EP | 1314301 A1 | 28-05-2003 |
| | | | WO | 0217602 A1 | 28-02-2002 |
| | | | GB | 2370728 A ,B | 03-07-2002 |
| | | | EP | 1366435 A2 | 03-12-2003 |
| | | | WO | 0217652 A2 | 28-02-2002 |
| | | | GB | 2371382 A ,B | 24-07-2002 |
| | | | US | 2004249846 A1 | 09-12-2004 |
| EP 1494190 | A | 05-01-2005 | CA | 2472668 A1 | 30-12-2004 |
| | | | EP | 1494190 A1 | 05-01-2005 |
| | | | US | 2005015862 A1 | 27-01-2005 |
| | | | US | 2005001727 A1 | 06-01-2005 |
| WO 03036247 | A | 01-05-2003 | EP | 1447653 A1 | 18-08-2004 |
| | | | WO | 03036247 A1 | 01-05-2003 |
| EP 1447653 | A | 18-08-2004 | EP | 1447653 A1 | 18-08-2004 |
| | | | WO | 03036247 A1 | 01-05-2003 |
| US 5507291 | A | 16-04-1996 | NONE | | |

EPO FORM P0459

For more details about this annex : see Official Journal of the European Patent Office, No. 12/82

15